# EUROPEAN PATENT APPLICATION

(11) **EP 4 082 595 A1**
(43) Date of publication of application: **02.11.2022**
(21) Application number: 19957758.6
(22) Date of filing: 25.12.2019
(51) Int. Cl.: A61M 25/00

(54) **MEDICAL LIQUID INJECTION DEVICE**

(71) Applicant: Asahi Intecc Co., Ltd., Seto-shi, Aichi 489-0071 (JP)
(72) Inventor: NAMIMA, Satoshi, Seto-shi, Aichi 489-0071 (JP); KUBO, Yuta, Seto-shi, Aichi 489-0071 (JP); ISHIKAWA, Masatomo, Seto-shi, Aichi 489-0071 (JP)
(74) Representative: Winter, Brandl - Partnerschaft mbB
(86) International application number: PCT/JP2019/050816
(87) International publication number: WO 2021/130896

(57) **Abstract**

Provided is a medical liquid injection device including a catheter shaft having a first lumen, and a connector being connected to a proximal end portion of the catheter shaft and including a second lumen communicating with the first lumen. The connector is formed with a weak portion extending from an outer peripheral surface of the connector to an inner peripheral surface of the connector and being weaker than another portion of the connector.

## Description

### TECHNICAL FIELD

The technique disclosed herein relates to a medical liquid injection device.

### BACKGROUND

For example, there is known a medical liquid injection device for injecting a medical liquid into a body of a patient by using a catheter that can be inserted into a living lumen such as a vascular system or a digestive system (see, for example, Patent Literatures 1 and 2).

In the medical liquid injection device described in Patent Literature 1, a medical liquid administration tube is connected to a connector connected to a proximal end portion of a sheath tube and a medical liquid is administered from the medical liquid administration tube, to inject the medical liquid into the body of the patient via the connector and the sheath tube. In the medical liquid injection device described in Patent Literature 2, a syringe is connected to a Y-connector connected to a proximal end portion of a tubular member included in a catheter and a medical liquid is administered from the syringe, to inject the medical liquid into the body of the patient via the Y-connector and the tubular member.

### CITATION LIST

### Patent Literature

Patent Literature 1: JP 2002-291902 A
Patent Literature 2: JP 2010-137099 A

### SUMMARY

### TECHNICAL PROBLEM

In such a conventional medical liquid injection device, after the injection of the medical liquid into the body of the patient is completed, there is a problem that a relatively large amount of the medical liquid remains in a medical liquid supply passage such as the medical liquid administration tube and the Y-connector, so that it is difficult to suppress wasteful use of the medical liquid.

The present specification discloses a technique by which it is possible to solve the above-described problems.

### SOLUTION TO PROBLEM

The techniques disclosed herein can be realized in the following aspects, for example.
(1) A medical liquid injection device disclosed herein is a medical liquid injection device and includes a catheter shaft including a first lumen, and a connector being connected to a proximal end portion of the catheter shaft and including a second lumen communicating with the first lumen, in which the connector is formed with a weak portion extending from an outer peripheral surface of the connector to an inner peripheral surface of the connector and being weaker than another portion of the connector.
   According to the medical liquid injection device, for example, when the weak portion is punctured by an injection needle of an injector, it is possible to obtain a state where a syringe of the injector and the second lumen formed in the connector communicate with each other via the injection needle. In this state, a medical liquid housed in the syringe can be directly supplied to the second lumen, and thus, the medical liquid can be supplied to the first lumen communicating with the second lumen. Therefore, according to the medical liquid injection device, as compared with a configuration in which a medical liquid administration tube is connected to a proximal end portion of the connector, a configuration in which a Y-connector is used instead of the connector and a syringe is connected to a branch pipe of the Y-connector, and the like, it is possible to reduce the amount of medical liquid remaining in a medical liquid supply passage after the injection of the medical liquid into a body of a patient is completed, and to prevent wasteful use of the medical liquid.
(2) The medical liquid injection device described above may have a configuration in which the second lumen includes a first portion and a second portion that is located closer to a proximal end side of the connector than the first portion and that has a larger inner diameter than the first portion, and the weak portion extends to a portion of the inner peripheral surface of the connector that defines the first portion of the second lumen. In the configuration of the medical liquid injection device, the weak portion extends to a portion of the inner peripheral surface of the connector that defines the first portion included in the second lumen, and thus, a medical liquid housed in a syringe is supplied to the first portion of the second lumen via an injection needle penetrating the weak portion. The first portion is located closer to the distal end side of the connector than the second portion, and thus, it is possible to prevent the medical liquid supplied to the first portion from flowing into the second portion having a larger diameter and thus a larger capacity than the first portion. According to the configuration of the medical liquid injection device, after the injection of the medical liquid into the body of the patient is completed, it is possible to prevent the medical liquid from remaining in the second portion having a relatively large capacity, and thus, it is possible to effectively suppress wasteful use of the medical liquid.
(3) The medical liquid injection device described above may have a configuration in which the weak portion is inclined with respect to a radial direction of the connector so that the weak portion is located closer to a distal end side of the connector as a distance from the inner peripheral surface of the connector decreases. According to the configuration of the medical liquid injection device, it is possible to supply a medical liquid housed in a syringe to a position closer to a distal end side of the second lumen, via an injection needle penetrating the weak portion, and as a result, the amount of the medical liquid remaining in the medical liquid supply passage can be strongly reduced, and wasteful use of the medical liquid can be effectively suppressed. According to the configuration of the medical liquid injection device, it is possible to prevent a distal end of the injection needle from passing through the second lumen and piercing an inner wall on the opposite side, and to improve the operability of the medical liquid injection device.
(4) The medical liquid injection device described above may have a configuration in which the connector includes a connector main body portion that is tubular and that includes the second lumen, the connector main body portion being formed with a communication hole opening on an outer peripheral surface of the connector main body portion and communicating with the second lumen, and the weak portion includes the communication hole and a sealing member that seals the communication hole. According to the configuration of the medical liquid injection device, the weak portion can be easily provided in the connector, and it is possible to suppress a decrease in the manufacturing efficiency of the medical liquid injection device due to the provision of the weak portion.
(5) The medical liquid injection device described above may have a configuration in which the communication hole is filled with the sealing member. According to the configuration of the medical liquid injection device, the adhesion between an injection needle penetrating the weak portion and the sealing member can be improved, and wasteful use of the medical liquid due to leakage of the medical liquid from the vicinity of the injection needle can be suppressed.
(6) The medical liquid injection device described above may have a configuration in which the communication hole is filled with the sealing member over the full length of the communication hole. According to the configuration of the medical liquid injection device, a contact area between an injection needle penetrating the weak portion and the sealing member can be increased, and wasteful use of the medical liquid due to leakage of the medical liquid from the vicinity of the injection needle can be effectively suppressed.
(7) The medical liquid injection device described above may have a configuration in which only a portion of the communication hole on a side of the outer peripheral surface of the connector main body portion is filled with the sealing member. According to the configuration of the medical liquid injection device, it is easier to adjust a position of an injection needle so that a distal end of the injection needle is prevented from passing through the second lumen and piercing an inner wall on the opposite side and a hole on the distal end of the injection needle is exposed from the sealing member, and thus, the operability of the medical liquid injection device can be improved.
(8) The medical liquid injection device described above may have a configuration in which the sealing member is arranged on the outer peripheral surface of the connector main body portion to cover the opening of the communication hole. According to the configuration of the medical liquid injection device, the communication hole can be more surely sealed by the sealing member, and it is possible to effectively suppress wasteful use of the medical liquid due to leakage of the medical liquid via the weak portion. Moreover, according to the configuration of the medical liquid injection device, it is easier to adjust a position of an injection needle so that a distal end of the injection needle is prevented from passing through the second lumen and piercing the inner wall on the opposite side and the hole on the distal end of the injection needle is exposed from the sealing member, and thus, the operability of the medical liquid injection device can be improved.
(9) The medical liquid injection device described above may have a configuration in which the sealing member is formed of a resin material. According to the configuration of the medical liquid injection device, it is possible to realize the weak portion that can be easily punctured by the injection needle, and to improve the operability of the medical liquid injection device. Moreover, according to the configuration of the medical liquid injection device, the adhesion between an injection needle penetrating the weak portion and the sealing member can be further improved, and wasteful use of the medical liquid due to leakage of the medical liquid from the vicinity of the injection needle can be effectively suppressed.

It is noted that the techniques disclosed herein can be realized in various aspects, for example, in aspects such as a medical liquid injection device and a manufacturing method thereof.

### BRIEF DESCRIPTION OF DRAWINGS

FIG. 1 is an explanatory diagram schematically illustrating a configuration of a medical liquid injection device 100 according to a first embodiment.
FIG. 2 is an explanatory diagram illustrating a method of using the medical liquid injection device 100 according to the first embodiment.
FIG. 3 is an explanatory diagram schematically illustrating a configuration of a medical liquid injection device 100a according to a second embodiment.
FIG. 4 is an explanatory diagram schematically illustrating a configuration of a medical liquid injection device 100b according to a third embodiment.

### EMBODIMENTS

### A. First Embodiment:

### A-1. Configuration of Medical Liquid Injection Device 100:

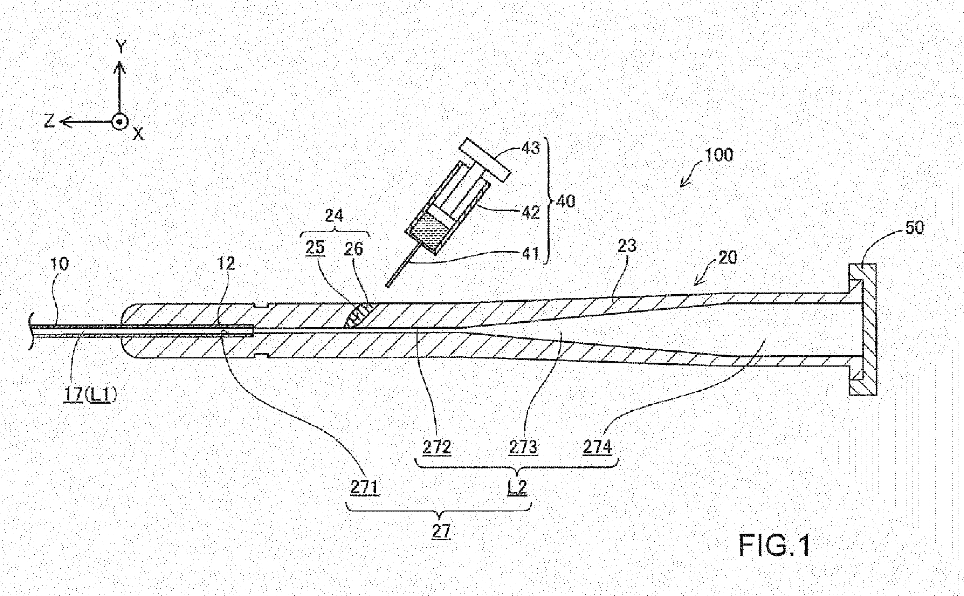

FIG. 1 is an explanatory diagram schematically illustrating a configuration of a medical liquid injection device 100 according to a first embodiment. FIG. 1 illustrates a configuration of a longitudinal cross section (YZ cross section) of the medical liquid injection device 100, together with XYZ axes orthogonal to each other. In FIG. 1, a Z-axis positive direction side is a distal end side (far side) to be inserted into a body, and a Z-axis negative direction side is a proximal end side (near side) operated by a technician such as a doctor. The same applies to the other drawings. In the present specification, in the medical liquid injection device 100 and each constituent member thereof, an end portion on a distal end side is referred to as "distal end", the distal end and a vicinity thereof are referred to as "distal end portion", an end portion on a proximal end side is referred to as "proximal end", and the proximal end and a vicinity thereof are referred to as "proximal end portion".

The medical liquid injection device 100 is a device for injecting a medical liquid into a body of a patient by using a catheter that can be inserted into a living lumen. The living lumen includes various lumens such as a vascular system, a lymph gland system, a biliary system, a urinary tract system, a respiratory system, a digestive system, a secretory gland, and a reproductive organ.

As illustrated in FIG. 1, the medical liquid injection device 100 includes a catheter shaft 10, a connector 20, and a cap member 50. In FIG. 1, a part of the catheter shaft 10 on the distal end side is not illustrated.

The catheter shaft 10 is a member to be inserted into a living lumen of a patient, and to be used for carrying a medical liquid to a desired position in the body of the patient and injecting the medical liquid at the desired position. The catheter shaft 10 is an elongated, tubular member formed with a through-hole 17 extending from the distal end to the proximal end in an axial direction. A space formed by the through-hole 17 functions as a lumen (hereinafter, referred to as "first lumen L1") for distributing the medical liquid. An outer shape of a transverse cross section (XY cross section) at each position of the catheter shaft 10 may take any shape, for example, a circular shape. An outer shape of a transverse cross section (XY cross section) at each position of the through-hole 17 may take any shape, for example, a circular shape. An outer diameter of the catheter shaft 10 is, for example, about 0.5 mm to 2.0 mm, and a diameter of the through-hole 17 (diameter of the first lumen L1) is, for example, about 0.3 mm to 1.5 mm. FIG. 1 illustrates a state where the catheter shaft 10 has a linear shape substantially parallel to the Z-axis direction, however, the catheter shaft 10 has enough flexibility to be curved. In the present embodiment, the catheter shaft 10 includes, at the distal end portion thereof, a needle portion (not illustrated) having a hollow shape, and a hollow portion of the needle portion forms a part of the first lumen L1.

The catheter shaft 10 is formed of a resin material or a metal material, for example. Examples of the resin material for forming the catheter shaft 10 may include a polyamide resin, a polyolefin resin, a polyester resin, a polyurethane resin, a silicone resin, and a fluororesin. Examples of the metal material for forming the catheter shaft 10 include stainless steel such as SUS304, a NiTi alloy, and a cobalt-chromium alloy. The entire catheter shaft 10 may be formed of the same material, or a part of the catheter shaft 10 may be formed of a material different from another part.

The connector (also referred to as "hub") 20 is a member connected to a proximal end portion 12 of the catheter shaft 10 and used for supplying the medical liquid to the catheter shaft 10. The connector 20 includes a connector main body portion 23. The connector main body portion 23 is a tubular member formed with a through-hole 27 extending from the distal end to the proximal end in the axial direction (Z-axis direction). An outer shape of the transverse cross section (XY cross section) at each position of the connector 20 may take any shape, for example, a circular shape. A shape of the transverse cross section (XY cross section) at each position of the through-hole 27 may take any shape, for example, a circular shape.

The connector main body portion 23 is formed of a resin material, for example. Examples of the resin material for forming the connector main body portion 23 include an acrylic resin, polyurethane, polypropylene, and unplasticized polyvinyl chloride. The entire connector main body portion 23 may be formed of the same material, or a part of the connector main body portion 23 may be formed of a material different from another part.

The through-hole 27 formed in the connector main body portion 23 includes a shaft housing portion 271, a small diameter portion 272, a tapered portion 273, and a large diameter portion 274, which are arranged consecutively from the distal end side to the proximal end side. These portions constituting the through-hole 27 are coaxial with each other and communicate with each other. A diameter of the shaft housing portion 271 is larger than a diameter of the small diameter portion 272, and a diameter of the large diameter portion 274 is larger than the diameter of the small diameter portion 272. The diameter of the tapered portion 273 is substantially the same as the diameter of the small diameter portion 272 at a boundary position with the small diameter portion 272, and is substantially the same as the diameter of the large diameter portion 274 at a boundary position with the large diameter portion 274, and thus, gradually increases from the boundary position with the small diameter portion 272 to the boundary position with the large diameter portion 274. The diameter of the small diameter portion 272 is, for example, about 0.5 mm to 1.0 mm, and the diameter of the large diameter portion 274 is, for example, about 0.8 mm to 2.0 mm. The small diameter portion 272 is an example of a first portion in the claims, and the tapered portion 273 and/or the large diameter portion 274 are an example of a second portion in the claims.

The proximal end portion 12 of the catheter shaft 10 is inserted into the shaft housing portion 271 of the through-hole 27 formed in the connector main body portion 23. A space formed by the small diameter portion 272, the tapered portion 273, and the large diameter portion 274 of the through-hole 27 functions as a lumen (hereinafter, referred to as "second lumen L2") for inserting a guide wire (not illustrated) and for distributing a medical liquid. The small diameter portion 272 of the through-hole 27 communicates with the first lumen L1 of the catheter shaft 10 inserted into the shaft housing portion 271. Therefore, the second lumen L2 including the small diameter portion 272, the tapered portion 273, and the large diameter portion 274 communicates with the first lumen L1 of the catheter shaft 10.

The cap member 50 is a lid-shaped member attached to the proximal end of the connector 20. When the cap member 50 is attached to the connector 20, the proximal end side of the second lumen L2 formed in the connector 20 is tightly closed. The cap member 50 is formed of a resin material, for example. Examples of the resin material for forming the cap member 50 include polyurethane, polypropylene, and unplasticized polyvinyl chloride.

### A-2. Configuration of Weak Portion 24:

In the medical liquid injection device 100 according to the present embodiment, the connector 20 is formed with a weak portion 24 that extends from an outer peripheral surface of the connector 20 to an inner peripheral surface of the connector 20 and is weaker than another portion of the connector 20. This aspect will be described in the following.

The connector main body portion 23 is formed with a communication hole 25 opening on an outer peripheral surface of the connector main body portion 23 and communicating with the second lumen L2. The communication hole 25 is inclined with respect to a radial direction (direction orthogonal to the axial direction) of the connector 20, to be located closer to the distal end side of the connector 20 as a distance from an inner peripheral surface of the connector main body portion 23 (distance in the direction orthogonal to the axial direction) decreases. The communication hole 25 communicates with the small diameter portion 272 of the second lumen L2. Across sectional shape orthogonal to a stretching direction of the communication hole 25 may take any shape, for example, a circular shape. The diameter of the communication hole 25 is about 0.3 mm to 1.0 mm, for example.

The communication hole 25 formed in the connector main body portion 23 is filled with a sealing member 26. That is, the sealing member 26 functions as a plug closing the communication hole 25. In the present embodiment, the sealing member 26 is filled over the full length of the communication hole 25. An outer peripheral surface of the sealing member 26 does not protrude radially outward and is not recessed radially inward from the outer peripheral surface of the connector main body portion 23. That is, the outer peripheral surface of the sealing member 26 forms a flat surface together with the outer peripheral surface of the connector main body portion 23. An inner peripheral surface of the sealing member 26 does not protrude radially inward and is not recessed radially outward from the inner peripheral surface of the connector main body portion 23. That is, the inner peripheral surface of the sealing member 26 forms a flat surface together with the inner peripheral surface of the connector main body portion 23. The outer peripheral surface of the sealing member 26 may be configured to protrude radially outward or to be recessed radially inward from the outer peripheral surface of the connector main body portion 23. The inner peripheral surface of the sealing member 26 may be configured to protrude radially inward or to be recessed radially outward from the inner peripheral surface of the connector main body portion 23. In the present embodiment, the sealing member 26 is formed of a resin material. Examples of the resin material for forming the sealing member 26 include urethane rubber, silicone rubber, and the like.

In the present embodiment, the material forming the sealing member 26 is a material that is more flexible than the material forming the connector main body portion 23. Thus, in the connector 20, a portion including the communication hole 25 and the sealing member 26 applied into the communication hole 25 is a portion (hereinafter, referred to as "weak portion 24") that is weaker than another portion of the connector 20. The weak portion 24 extends from the outer peripheral surface of the connector 20 to the inner peripheral surface of the connector 20.

As described above, the communication hole 25 forming the weak portion 24 is inclined with respect to the radial direction of the connector 20 to be located closer to the distal end side of the connector 20 as the distance from the inner peripheral surface of the connector main body portion 23 decreases, and thus, the weak portion 24 is also similarly inclined. The communication hole 25 forming the weak portion 24 communicates with the small diameter portion 272 included in the second lumen L2, and thus, the weak portion 24 extends to a portion of the inner peripheral surface of the connector 20 that defines the small diameter portion 272 included in the second lumen L2.

### A-3. Action of Medical Liquid Injection Device 100:

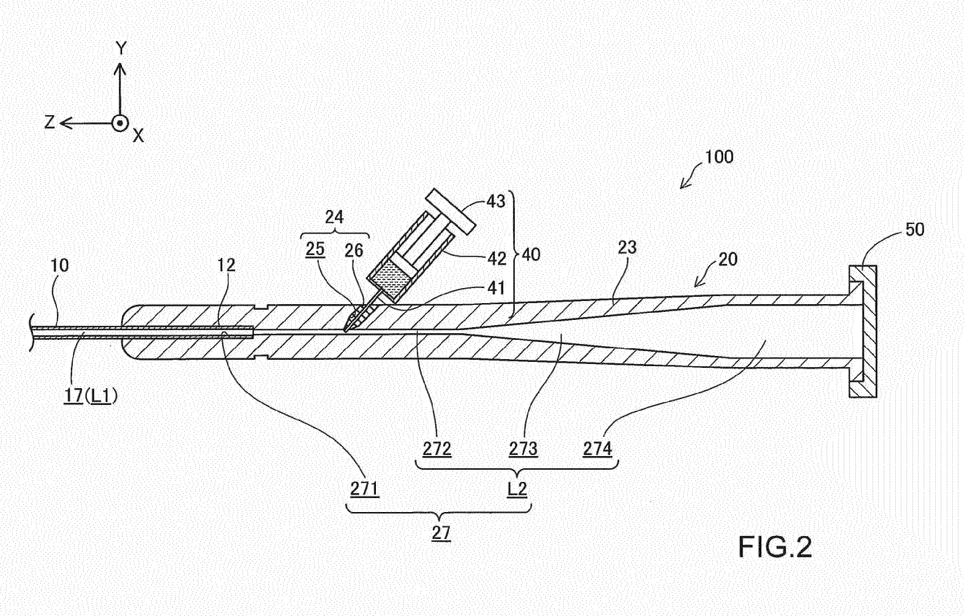

A method of using the medical liquid injection device 100 according to the present embodiment will be described below. For example, in using the medical liquid injection device 100, the distal end portion (needle portion) of the catheter shaft 10 is guided by a guide wire or the like inserted into the first lumen L1 formed in the connector 20, to be moved to a target position in a body of a patient (position where the medical liquid is to be administered). The cap member 50 is attached to the proximal end of the connector 20, to tightly close the proximal end side of the second lumen L2. In this state, as illustrated in FIG. 2, an injector 40 including a syringe 42 housing a medical liquid, a plunger 43, and an injection needle 41 is prepared, and an operator punctures the weak portion 24 of the connector 20 with the injection needle 41 of the injector 40. At this time, the operator pushes and advances the injection needle 41 until a distal end of the injection needle 41 penetrates the sealing member 26 applied into the communication hole 25 and reaches the second lumen L2 (the small diameter portion 272 of the through-hole 27). As described above, the sealing member 26 is formed of a flexible resin material such as urethane rubber or silicone rubber, and thus, the operator can easily puncture the sealing member 26 with the injection needle 41. After being punctured, the sealing member 26 elastically deforms and adheres to an outer peripheral surface of the injection needle 41, and as a result, the periphery of the injection needle 41 is sealed. When the injection needle 41 is removed from the sealing member 26, a through-hole opened in the sealing member 26 by the injection needle 41 is closed by the elastic deformation of the sealing member 26, and as a result, the communication hole 25 is again sealed by the sealing member 26.

In a state where the distal end of the injection needle 41 penetrates the sealing member 26 and is inserted up to the second lumen L2, the operator pushes the plunger 43 of the injector 40 to discharge the medical liquid housed in the syringe 42 into the second lumen L2 of the connector 20. The proximal end side of the second lumen L2 is tightly closed by the cap member 50, and thus, the medical liquid supplied to the second lumen L2 does not enter a portion on the proximal end side from the supply point (that is, a portion including the tapered portion 273 and the large diameter portion 274 of the through-hole 27), and advances toward the first lumen L1 of the catheter shaft 10 communicating with the second lumen L2. Subsequently, the medical liquid passes through the first lumen L1 to reach the needle portion (not illustrated) arranged at the distal end portion of the catheter shaft 10, and is injected to a desired position in the body of the patient via the needle portion.

### A-4. Effects of First Embodiment:

As described above, the medical liquid injection device 100 according to the present embodiment includes the catheter shaft 10 including the first lumen L1, and the connector 20 being connected to the proximal end portion 12 of the catheter shaft 10 and including the second lumen L2 communicating with the first lumen L1. The connector 20 is formed with the weak portion 24 that extends from the outer peripheral surface of the connector 20 to the inner peripheral surface of the connector 20 and is weaker than another portion of the connector 20. Therefore, in the medical liquid injection device 100 according to the present embodiment, when the weak portion 24 is punctured by the injection needle 41 of the injector 40, it is possible to obtain a state where the syringe 42 of the injector 40 and the second lumen L2 formed in the connector 20 communicate with each other via the injection needle 41. In this state, the medical liquid housed in the syringe 42 can be directly supplied to the second lumen L2, and thus, the medical liquid can be supplied to the first lumen L1 communicating with the second lumen L2. Therefore, according to the medical liquid injection device 100 in the present embodiment, as compared with a configuration in which a medical liquid administration tube is connected to a proximal end portion of the connector 20, a configuration in which a Y-connector is used instead of the connector 20 and a syringe is connected to a branch pipe of the Y-connector, and the like, it is possible to reduce the amount of medical liquid remaining in a medical liquid supply passage after the injection of the medical liquid into the body of the patient is completed, and to prevent wasteful use of the medical liquid.

In the medical liquid injection device 100 according to the present embodiment, the second lumen L2 formed in the connector 20 includes the small diameter portion 272 and a portion (the large diameter portion 274 and the tapered portion 273) located on the proximal end side of the connector 20 with respect to the small diameter portion 272 and having an inner diameter larger than an inner diameter of the small diameter portion 272. The weak portion 24 formed in the connector 20 extends to a portion of the inner peripheral surface of the connector 20 that defines the small diameter portion 272 included in the second lumen L2. As described above, in the medical liquid injection device 100 according to the present embodiment, the weak portion 24 extends to the portion of the inner peripheral surface of the connector 20 that defines the small diameter portion 272 included in the second lumen L2, and thus, the medical liquid housed in the syringe 42 is supplied to the small diameter portion 272 included in the second lumen L2, via the injection needle 41 penetrating the weak portion 24. The small diameter portion 272 is located on the distal end side of the connector 20 with respect to the large diameter portion 274 and the tapered portion 273, so that it is possible to prevent the medical liquid supplied to the small diameter portion 272 from flowing into the large diameter portion 274 and the tapered portion 273 that have a larger diameter and thus a larger capacity than the small diameter portion 272. According to the medical liquid injection device 100 in the present embodiment, after the injection of the medical liquid into the body of the patient is completed, it is possible to prevent the medical liquid from remaining in the large diameter portion 274 and the tapered portion 273 that have a relatively large capacity, and thus, it is possible to effectively suppress wasteful use of the medical liquid.

In the medical liquid injection device 100 according to the present embodiment, the weak portion 24 formed in the connector 20 is inclined with respect to the radial direction of the connector 20, so that the weak portion 24 is located closer to the distal end side of the connector 20 as the distance from the inner peripheral surface of the connector 20 decreases. Therefore, according to the medical liquid injection device 100 in the present embodiment, it is possible to supply the medical liquid housed in the syringe 42 to a position closer to the distal end side of the second lumen L2, via the injection needle 41 penetrating the weak portion 24, and as a result, the amount of the medical liquid remaining in the medical liquid supply passage can be strongly reduced, and wasteful use of the medical liquid can be effectively suppressed. Moreover, according to the medical liquid injection device 100 in the present embodiment, it is possible to prevent the distal end of the injection needle 41 from passing through the second lumen L2 and piercing an inner wall (inner peripheral surface) on the opposite side, and to improve the operability of the medical liquid injection device 100.

In the medical liquid injection device 100 according to the present embodiment, the connector 20 includes the connector main body portion 23 having a tubular shape and including the second lumen L2. The connector main body portion 23 is formed with the communication hole 25 that opens on the outer peripheral surface of the connector main body portion 23 and communicates with the second lumen L2. The weak portion 24 is includes the communication hole 25 and the sealing member 26 for sealing the communication hole 25. Therefore, according to the medical liquid injection device 100 in the present embodiment, the weak portion 24 can be easily provided in the connector 20, and it is possible to suppress a decrease in the manufacturing efficiency of the medical liquid injection device 100 due to the provision of the weak portion 24.

In the medical liquid injection device 100 according to the present embodiment, the communication hole 25 formed in the connector 20 is filled with the sealing member 26. Therefore, according to the medical liquid injection device 100 in the present embodiment, the adhesion between the injection needle 41 penetrating the weak portion 24 and the sealing member 26 can be improved, and wasteful use of the medical liquid due to leakage of the medical liquid from the vicinity of the injection needle 41 can be suppressed. In particular, in the medical liquid injection device 100 according to the present embodiment, the communication hole 25 formed in the connector 20 is filled with the sealing member 26 over the full length of the communication hole 25, and thus, a contact area between the injection needle 41 penetrating the weak portion 24 and the sealing member 26 can be increased, so that it is possible to effectively suppress wasteful use of the medical liquid due to leakage of the medical liquid from the vicinity of the injection needle 41.

In the medical liquid injection device 100 according to the present embodiment, the sealing member 26 is formed of a resin material. Therefore, according to the medical liquid injection device 100 in the present embodiment, it is possible to realize the weak portion 24 that can be easily punctured by the injection needle 41, and to improve the operability of the medical liquid injection device 100. Moreover, according to the medical liquid injection device 100 in the present embodiment, the adhesion between the injection needle 41 penetrating the weak portion 24 and the sealing member 26 can be further improved, and wasteful use of the medical liquid due to leakage of the medical liquid from the vicinity of the injection needle 41 can be effectively suppressed.

### B. Second Embodiment:

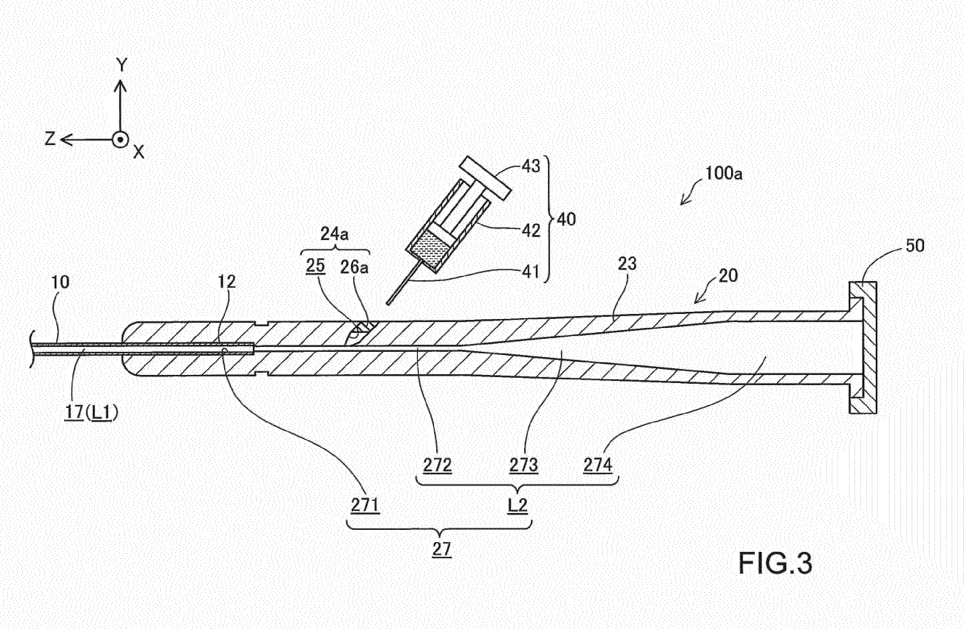

FIG. 3 is an explanatory diagram schematically illustrating a configuration of a medical liquid injection device 100a according to a second embodiment. In the following, a configuration the same as that of the medical liquid injection device 100 of the first embodiment described above in the configuration of the medical liquid injection device 100a of the second embodiment will be referred to by the same reference numerals, and a description thereof will be omitted where appropriate.

As illustrated in FIG. 3, in the medical liquid injection device 100a according to the second embodiment, a configuration of a weak portion 24a is different from the configuration of the weak portion 24 of the medical liquid injection device 100 of the first embodiment. More specifically, in the medical liquid injection device 100a according to the second embodiment, only a portion of the communication hole 25 on a side of the outer peripheral surface of the connector main body portion 23 is filled with a sealing member 26a included in the weak portion 24a. Also in this case, similarly to the first embodiment, an outer peripheral surface of the sealing member 26a does not protrude radially outward and is not recessed radially inward from the outer peripheral surface of the connector main body portion 23. That is, the outer peripheral surface of the sealing member 26a forms a flat surface together with the outer peripheral surface of the connector main body portion 23. The outer peripheral surface of the sealing member 26a may be configured to protrude radially outward or to be recessed radially inward from the outer peripheral surface of the connector main body portion 23.

As described above, in the medical liquid injection device 100a according to the second embodiment, only a portion of the communication hole 25 on a side of the outer peripheral surface of the connector main body portion 23 is filled with the sealing member 26a included in the weak portion 24a. Therefore, according to the medical liquid injection device 100a in the second embodiment, it is easier to adjust the position of the injection needle 41 so that the distal end of the injection needle 41 is prevented from passing through the second lumen L2 and piercing the inner wall (inner peripheral surface) on the opposite side, and a hole on the distal end of the injection needle 41 is exposed from the sealing member 26a, and thus, the operability of the medical liquid injection device 100a can be improved.

### C. Third Embodiment:

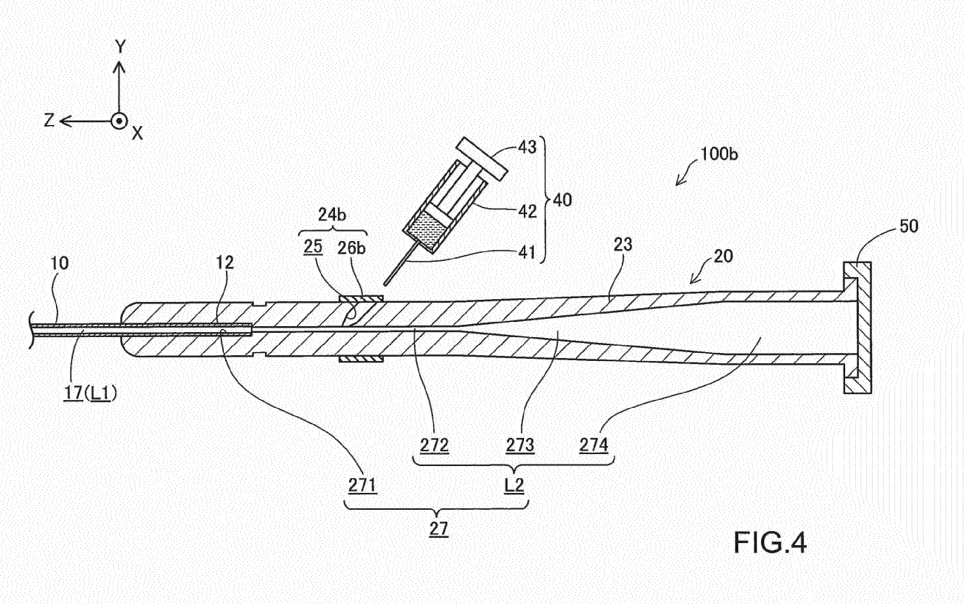

FIG. 4 is an explanatory diagram schematically illustrating a configuration of a medical liquid injection device 100b according to a third embodiment. In the following, a configuration the same as that of the medical liquid injection device 100 of the first embodiment described above in the configuration of the medical liquid injection device 100b of the third embodiment will be referred to by the same reference numerals, and a description thereof will be omitted where appropriate.

As illustrated in FIG. 4, in the medical liquid injection device 100b according to the third embodiment, a configuration of a weak portion 24b is different from the configuration of the weak portion 24 of the medical liquid injection device 100 of the first embodiment. More specifically, in the medical liquid injection device 100b according to the third embodiment, a sealing member 26b included in the weak portion 24b is formed by an O-ring wound around the outer peripheral surface of the connector 20. That is, the communication hole 25 is not filled with the sealing member 26b included in the weak portion 24b, but the sealing member 26b is arranged on the outer peripheral surface of the connector main body portion 23 to cover an opening of the communication hole 25.

As described above, in the medical liquid injection device 100b according to the third embodiment, the sealing member 26b included in the weak portion 24b is arranged on the outer peripheral surface of the connector main body portion 23 to cover the opening of the communication hole 25. Therefore, according to the medical liquid injection device 100b in the third embodiment, the communication hole 25 can be more surely sealed by the sealing member 26b, and it is possible to effectively suppress wasteful use of the medical liquid due to leakage of the medical liquid via the weak portion 24b. Moreover, according to the medical liquid injection device 100b in the third embodiment, it is easier to adjust the position of the injection needle 41 so that the distal end of the injection needle 41 is prevented from passing through the second lumen L2 and piercing the inner wall (inner peripheral surface) on the opposite side, and the hole on the distal end of the injection needle 41 is exposed from the sealing member 26b, and thus, the operability of the medical liquid injection device 100b can be improved.

### D. Modifications:

The techniques disclosed herein are not limited to the above-described embodiments, and may be modified into various modes without departing from the spirit of the above-described embodiments. For example, the following modifications can be applied.

The configuration of the medical liquid injection device 100 in each of the above embodiments is merely an example, and various modifications are possible. For example, in each of the above-described embodiments, the weak portion 24 is inclined with respect to the radial direction of the connector 20, so that the weak portion 24 is located closer to the distal end side of the connector 20 as the distance from the inner peripheral surface of the connector 20 decreases. However, the weak portion 24 may be inclined in an orientation different from the orientation described above, or the weak portion 24 may extend in parallel to the radial direction of the connector 20.

In each of the above-described embodiments, the weak portion 24 extends to a portion of the inner peripheral surface of the connector 20 that defines the small diameter portion 272 of the second lumen L2. However, the weak portion 24 may extend to a portion of the inner peripheral surface of the connector 20 that defines other portion of the second lumen L2 (the tapered portion 273 or the large diameter portion 274). A shape of the through-hole 27 can be freely changed, and for example, the tapered portion 273 may be omitted, or the diameter of the through-hole 27 may be substantially constant in the axial direction.

In each of the above-described embodiments, the weak portion 24 includes the communication hole 25 formed in the connector main body portion 23 and the sealing member 26 for sealing the communication hole 25, but the configuration of the weak portion 24 is not limited thereto. For example, the weak portion 24 may be a portion where a part of the connector main body portion 23 is weakened by performing a predetermined process, or a portion where a thickness of a part of the connector main body portion 23 is extremely thin.

The medical liquid injection device 100 in each of the above-described embodiments has a configuration in which one weak portion 24 is provided. However, two or more weak portions 24 may be provided. Specifically, two or more of the weak portions 24 may be provided along a circumferential direction of the connector 20 or along the axial direction.

In each of the above-described embodiments, the cap member 50 is attached to the proximal end of the connector 20, however, a Y-connector or a syringe may be attached to the proximal end of the connector 20. In each of the above-described embodiments, the weak portion 24 is configured to be punctured by the injection needle 41. However, the weak portion 24 may be configured to be punctured by an instrument other than the injection needle 41.

The material of each member included in the medical liquid injection device 100 in each of above-described embodiments is merely an example, and various modifications are possible.

### DESCRIPTION OF REFERENCE NUMERALS

10: Catheter shaft
12: Proximal end portion
17: Through-hole
20: Connector
23: Connector main body portion
24: Weak portion
25: Communication hole
26, 26a, 26b: Sealing member
27: Through-hole
40: Injector
41: Injection needle
42: Syringe
43: Plunger
50: Cap member
100, 100a, 100b: Medical liquid injection device
271: Shaft housing portion
272: Small diameter portion
273: Tapered portion
274: Large diameter portion
L1: First lumen
L2: Second lumen

## Claims

1. A medical liquid injection device, comprising:
a catheter shaft including a first lumen; and
a connector being connected to a proximal end portion of the catheter shaft and including a second lumen communicating with the first lumen, wherein
the connector is formed with a weak portion extending from an outer peripheral surface of the connector to an inner peripheral surface of the connector and being weaker than another portion of the connector.

2. The medical liquid injection device according to claim 1, wherein
the second lumen includes a first portion and a second portion that is located closer to a proximal end side of the connector than the first portion and that has a larger inner diameter than the first portion, and
the weak portion extends to a specific portion of the inner peripheral surface of the connector, the specific portion defining the first portion of the second lumen.

3. The medical liquid injection device according to claim 1 or 2, wherein
the weak portion is inclined with respect to a radial direction of the connector so that the weak portion is located closer to a distal end side of the connector as a distance from the inner peripheral surface of the connector decreases.

4. The medical liquid injection device according to any one of claims 1 to 3, wherein
the connector includes a connector main body portion that is tubular and that includes the second lumen, the connector main body portion being formed with a communication hole opening on an outer peripheral surface of the connector main body portion and communicating with the second lumen, and
the weak portion includes the communication hole and a sealing member that seals the communication hole.

5. The medical liquid injection device according to claim 4, wherein
the communication hole is filled with the sealing member.

6. The medical liquid injection device according to claim 5, wherein
the communication hole is filled with the sealing member over the full length of the communication hole.

7. The medical liquid injection device according to claim 5, wherein
only a portion of the communication hole on a side of the outer peripheral surface of the connector main body portion is filled with the sealing member.

8. The medical liquid injection device according to claim 4, wherein
the sealing member is arranged on the outer peripheral surface of the connector main body portion to cover the opening of the communication hole.

9. The medical liquid injection device according to any one of claims 4 to 8, wherein
the sealing member is formed of a resin material.
